# EUROPEAN PATENT SPECIFICATION

(11) **EP 2 671 579 B1**
(45) Date of publication and mention of the grant of the patent: **08.02.2017**
(21) Application number: 11857733.7
(22) Date of filing: 15.12.2011
(51) Int. Cl.: A61K 31/23, A23L 2/52, A61K 31/231, A61K 31/232, A61K 31/685, A61K 31/7024, A61P 3/10, A61P 43/00, A61K 9/10

(54) **FAT AND OIL COMPOSITION FOR PROMOTING INSULIN SECRETION**
FETT- UND ÖLZUSAMMENSETZUNG ZUR FÖRDERUNG DER INSULINSEKRETION
COMPOSITION D'HUILE ET DE GRAISSE DESTINÉE À PROMOUVOIR LA SÉCRÉTION D'INSULINE

(30) Priority: 31.01.2011 JP 2011018534
(43) Date of publication of application: 11.12.2013
(73) Proprietor: The Nisshin OilliO Group, Ltd., Tokyo 104-8285 (JP)
(72) Inventor: AOYAMA, Toshiaki, Yokosuka-shi Kanagawa 239-0832 (JP); TERADA, Shin, Yokosuka-shi Kanagawa 239-0832 (JP)
(74) Representative: KATZAROV S.A.
(86) International application number: PCT/JP2011/079021
(87) International publication number: WO 2012/105130

(56) References cited:
- EP-A1- 2 554 166
- WO-A1-2004/022050
- WO-A1-2010/149170
- WO-A1-2011/122389
- JP-A- 2000 309 794
- JP-A- 2002 504 501
- SHIN TERADA ET AL: "Dietary intake of medium- and long-chain triacylglycerols ameliorates insulin resistance in rats fed a high-fat diet", NUTRITION, ELSEVIER INC, US, vol. 28, no. 1, 30 April 2011 (2011-04-30) , pages 92-97, XP028391928, ISSN: 0899-9007, DOI: 10.1016/J.NUT.2011.04.008 [retrieved on 2011-05-12]
- KASAI, M. ET AL.: 'Effect of dietary medium- and long-chain triacylglycerols (MLCT) on accumulation of body fat in healthy humans' ASIA PAC. J. CLIN. NUTR. vol. 12, no. 2, 2003, pages 151 - 160, XP055116187
- IDE, H.: 'Stimulation of insulin secretion and hypoglycemia by medium chain triglyceride' NIHON NAIKA GAKKAI ZASSHI vol. 60, no. 2, 1971, pages 115 - 124, XP055116190
- JIANG, Z.M. ET AL.: 'A comparison of medium-chain and long-chain triglycerides in surgical patients' ANN. SURG. vol. 217, no. 2, 1993, pages 175 - 184, XP055116191
- FIACCADORI, E. ET AL.: 'Hemodynamic, respiratory, and metabolic effects of medium-chain triglyceride-enriched lipid emulsions following valvular heart surgery' CHEST vol. 106, no. 6, 1994, pages 1660 - 1667, XP055116192
- CHAMBRIER, C. ET AL.: 'Medium- and long-chain triacylglycerols in postoperative patients: structured lipids versus a physical mixture' NUTRITION vol. 15, no. 4, 1999, pages 274 - 277, XP055116198

## Description

### TECHNICAL FIELD

The present invention relates to an oil or fat composition as defined in the claims for use in treating or preventing diabetes, and more specifically, relates to an oil or fat composition for use in treating or preventing diabetes that includes a certain triacylglycerol, and a certain emulsifier as active ingredients.

### BACKGROUND ART

Diabetes is classified broadly into type I diabetes (insulin-dependent diabetes) and type II diabetes (noninsulin-dependent diabetes). The majority of diabetes from which Japanese suffer is type II diabetes. The disease develops through the progress of conditions such as insulin secretion failure that causes a decrease in the amount of insulin secretion, and insulin resistance that causes an impairment of an action of insulin of lowering a blood glucose level. These conditions are significantly related to genetic factors, as well as environmental factors such as unbalanced diet, excessive eating, insufficient exercise and stress.

In recent years, the number of patients with type II diabetes has increased, and already reportedly reaches twenty million including potential diabetic patients who may be referred to as borderline. Therefore, not only care of diabetic patients but also prevention of transition from potential diabetic patients to diabetic patients has been an important issue.

In general, a treatment of type II diabetes includes a pharmacological therapy carried out on the basis of an alimentary therapy and an exercise therapy. In pharmacological therapies for type II diabetes in Japan, a sulfonylurea agent (SU agent) that promotes insulin secretion has been frequently used because Japanese people have an insulin secretory capacity lower than that of Westerners. However, an SU agent is accompanied by problems of side effects such as exhaustion of pancreatic β cells, low blood glucose, pruritus, rash and body weight gain. Since it is necessary to perform a pharmacological therapy for diabetes consecutively for a long period of time, development of a promoting agent of insulin secretion for which a side effect is not concerned even if dosed on a continuous basis has been desired.

As such promoting agents of insulin secretion, for example, one containing an extraction liquid obtained from a skin portion of tuberous root of Ipomoea Batatas sp. as an active ingredient was reported in Patent Document 1.

Patent Document 1: Japanese Patent Publication No. 3677007.

WO2010149170 A1 (UNIV KOEBENHAVN) relates to 1 or 2 C₁₆-C₁₈ acyl glycerol based compounds which are capable of activating G-protein coupled receptor 119 and thereby stimulate GLP-1 release. Those compounds are useful in the prophylaxis and/or treatment of metabolic disorders and complications thereof, such as, type 2 diabetes mellitus (T2DM), obesity, insulin resistance, and cardiovascular disease.

### DISCLOSURE OF THE INVENTION

### Problems to be Solved by the Invention

The present invention was made in view of the foregoing circumstances, and an object of the invention is to provide an oil or fat composition as defined in the claims for use in treating or preventing diabetes, the composition being very safe, devoid of concerns of side effects even when ingested continuously, and effective in preventing diabetes and other uses.

### Means for Solving the Problems

The present inventor thoroughly investigated in order to solve the foregoing problems, and consequently found that the problems can be solved by an oil or fat composition for use in treating or preventing diabetes, the composition containing a triacylglycerol that includes a medium-chain fatty acid and a long-chain fatty acid each having a specific number of carbon atoms in its molecule as constituent fatty acids, and a certain emulsifier can solve the problems described above. Accordingly the present invention was completed.

Specifically, aspects of the present invention provide the following.

According to a first aspect of the present invention, an oil or fat composition use in treating or preventing diabetes is characterized by including a triacylglycerol and an emulsifier as active ingredients, the triacylglycerol having one or two medium-chain fatty acid residue(s) in a molecule and containing as constituent fatty acids a medium-chain fatty acid having 6 to 12 carbon atoms and a long-chain fatty acid having 16 to 24 carbon atoms, and the emulsifier being a polyglycerol fatty acid ester wherein a mass ratio of the triacylglycerol to the emulsifier in the oil or fat composition is 99.5:0.5 to 98:2.

According to a second aspect of the present invention, the constituent fatty acids of the triacylglycerol include a medium-chain fatty acid having 6 to 12 carbon atoms and a long-chain fatty acid having 16 to 24 carbon atoms in the oil or fat composition for use in treating or preventing diabetes of the first aspect.

According to a third aspect of the present invention, the medium-chain fatty acid is at least one selected from the group consisting of a saturated fatty acid having 8 and/or 10 carbon atoms in the oil or fat composition for use in treating or preventing diabetes of the first or second aspect.

According to a fourth aspect of the present invention, a food or drink composition contains the oil or fat composition for use of any one of the first to third aspects.

### Effects of the Invention

Since the oil or fat composition of the present invention is superior in an action for use in treating or preventing diabetes, it is particularly effective in the prevention or treatment of type II diabetes. In addition, since the active ingredients are a triacylglycerol and an emulsifier that may be ingested as food according to the oil or fat composition for use of the present invention, the composition for use is very safe, devoid of concerns of side effects; therefore, it can be used on a continuous basis reliably.

### BRIEF DESCRIPTION OF THE DRAWINGS

Fig. 1 shows a drawing illustrating results of determination of plasma insulin level in an (MLCT + emulsifier) administration trial.
Fig. 2 shows a drawing illustrating results of determination of plasma insulin level in an (LCT + emulsifier) administration trial.
Fig. 3 shows a drawing illustrating results of determination of plasma insulin level in an MLCT administration trial.
Fig. 4 shows a drawing illustrating results of determination of plasma insulin level in an (MCT + LCT) administration trial.
Fig. 5 shows a drawing illustrating results of determination of ((A): casual blood glucose level; and (B): plasma insulin level) in an (MLCT + emulsifier) administration trial.

### PREFERRED MODE FOR CARRYING OUT THE INVENTION

Hereinafter, specific embodiments of the present invention will be explained in detail.

The oil or fat composition for use in treating or preventing diabetes of the present invention includes a triacylglycerol and an emulsifier as active ingredients. Each component included in the oil or fat composition for use of the present invention will be specifically explained below.

### <Triacylglycerol>

The triacylglycerol that is one of the active ingredients of the oil or fat composition for use in treating or preventing diabetes (hereinafter, referred to as "oil or fat composition") of the present invention has one or two medium-chain fatty acid residue(s) in a molecule and includes as constituent fatty acids a medium-chain fatty acid having 6 to 12 carbon atoms and a long-chain fatty acid having 16 to 24 carbon atoms. More specifically, the triacylglycerol can be in the form of: a medium-chain fatty acid triacylglycerol (hereinafter, referred to as MCT) that includes only a medium-chain fatty acid as a constituent fatty acid per molecule of the triacylglycerol; a long-chain fatty acid triacylglycerol (hereinafter, referred to as LCT) that includes only a long-chain fatty acid; and a medium-chain and long-chain fatty acid triacylglycerol (hereinafter, referred to as M·LCT) that includes a medium-chain fatty acid and a long-chain fatty acid. However, the oil or fat composition for use of the present invention contains as one of active ingredients M·LCT that includes a medium-chain fatty acid having 6 to 12 carbon atoms and a long-chain fatty acid having 16 to 24 carbon atoms in a molecule of the triacylglycerol, as constituent fatty acids.

The oil or fat composition for use of the present invention may also include the MCT and/or LCT in addition to the M·LCT as an active ingredient. It is to be noted that such a triacylglycerol is herein referred to as a medium-chain and long-chain fatty acid triacylglycerol mixture (hereinafter, referred to as MLCT), and is expressed distinctively from M·LCT of a triacylglycerol having one or two medium-chain fatty acid residue(s) per molecule, and including a medium-chain fatty acid and a long-chain fatty acid as constituent fatty acids.

The triacylglycerol as an active ingredient includes a medium-chain fatty acid having 6 to 12 carbon atoms and a long-chain fatty acid having 16 to 24 carbon atoms, and preferably consists of a medium-chain fatty acid having 6 to 12 carbon atoms and a long-chain fatty acid having 16 to 24 carbon atoms. Examples of the medium-chain fatty acid having 6 to 12 carbon atoms include n-hexanoic acid, n-heptanoic acid, n-octanoic acid, n-nonanoic acid, n-decanoic acid, and lauric acid. The medium-chain fatty acid having 6 to 12 carbon atoms is preferably a saturated fatty acid having an even number of carbon atoms, and more preferably a saturated fatty acid having 8 and/or 10 carbon atoms (i.e., n-octanoic acid and/or n-decanoic acid). The medium-chain fatty acid can be obtained by hydrolyzing coconut oil, palm kernel oil or the like.

Examples of the long-chain fatty acid having 16 to 24 carbon atoms include long-chain saturated fatty acids such as palmitic acid, stearic acid, arachidic acid, behenic acid and lignoceric acid, long chain unsaturated fatty acids such as palmitoleic acid, oleic acid, linoleic acid, linolenic acid, γ-linolenic acid, arachidonic acid and icosapentaenoic acid. The long-chain fatty acid can be obtained by hydrolyzing soybean oil, rapeseed oil, corn oil, rice bran oil, sesame oil, cotton seed oil, sunflower seed oil, safflower oil, linseed oil, perilla oil, olive oil or the like produced by pressing and purifying a raw oil material for compression.

The triacylglycerol added as an active ingredient of the oil or fat composition for use of the present invention contains the medium-chain fatty acid and the long-chain fatty acid as constituent fatty acids, but the binding site of these fatty acids is not particularly limited.

The method for producing a triacylglycerol is not particularly limited, and for example, a conventionally well-known transesterification reaction or esterification reaction may be exemplified. The transesterification reaction may include, for example, a chemical transesterification reaction carried out using an inorganic catalyst such as sodium methoxide, and an enzymatic transesterification reaction carried out using lipase or the like. In the present invention, the transesterification reaction may be either chemical or enzymatic. In addition, the transesterification reaction is not particularly limited, which may be either a selective transesterification reaction or a nonselective

### transesterification reaction.

The esterification reaction may include, for example, an esterification reaction carried out using glycerol and a fatty acid, an esterification reaction carried out using glycerol and a fatty acid ester, and the like. In the present invention, the esterification reaction may be any of these reactions.

In the oil or fat composition for use of the present invention, the proportion of the medium-chain fatty acid in the entire constituent fatty acids of the triacylglycerol added as an active ingredient is preferably 15 to 40% by mass, and more preferably 20 to 35% by mass. When the proportion falls within this range, the action of promoting insulin secretion can be more effectively exhibited. It is to be noted that as a method for determining the proportion of the medium-chain fatty acid in the constituent fatty acids, for example, a method including: isolating the triacylglycerol from the oil or fat composition for use of the present invention by molecular distillation, column chromatography or the like; thereafter subjecting the constituent fatty acids to methyl esterification; and analyzing the product by gas chromatography.

The triacylglycerol, and constituent fatty acids thereof, i.e., the medium-chain fatty acid and the long-chain fatty acid are broadly present in nature, and also included in natural products to be used for eating, being very safe and devoid of a known side effect that may matter. According to the oil or fat composition for use of the present invention,
by selecting the triacylglycerol as an active ingredient, a side effect resulting from continuous ingestion can be avoided.

### <Emulsifier>

The emulsifier as one of the active ingredients of the oil or fat composition for use of the present invention is a polyglycerol fatty acid ester wherein a mass ratio of the triacylglycerol to the emulsifier in the oil or fat composition is 99.5:0.5 to 98:. The emulsifier is the polyglycerol fatty acid ester most superior in its action of promoting insulin secretion when used in combination with the triacylglycerol.

### (Polyglycerol Fatty Acid Ester)

In the oil or fat composition for use of the present invention, a polyglycerol fatty acid ester is contained as the emulsifier that is an active ingredient. Although the polyglycerol fatty acid ester is not particularly limited in the oil or fat composition for use of the present invention, in light of the effect of promoting insulin secretion being remarkable, the polyglycerol fatty acid ester has an HLB of preferably 4 to 15, and more preferably 4 to 9. The HLB value as referred to in connection with the oil or fat composition for use of the present invention means in the case in which at least two types of polyglycerol fatty acid esters having different HLB are included, a mean HLB value of these.

In the oil or fat composition for use of the present invention, the fatty acid constituting the polyglycerol fatty acid ester is not particularly limited, and is a saturated and/or unsaturated fatty acid having carbon atoms of typically 6 to 24, preferably 14 to 20, and more preferably 16 to 20. Specific examples of the fatty acid include caproic acid (n-hexanoic acid), caprylic acid (n-octanoic acid), capric acid (n-decanoic acid), lauric acid, myristic acid, palmitic acid, palmitoleic acid, stearic acid, oleic acid, linoleic acid, linolenic acid, arachidic acid, arachidonic acid, behenic acid, erucic acid, lignoceric acid, and the like.

In the oil or fat composition for use of the present invention, either a commercially available polyglycerol fatty acid ester, or a polyglycerol fatty acid ester produced by a conventionally well-known method may be used. Alternatively, a mixture of two or more types of these may be used. The method for producing the polyglycerol fatty acid ester is not particularly limited, and a conventionally well-known method such as, for example, esterification of glycerol with a fatty acid as well as transesterification of glycerol with an oil or fat may be exemplified. One example of the method for producing a polyglycerol fatty acid ester is shown in the following, but not limited thereto. First, glycerol is mixed with sodium hydroxide as a catalyst, and dried while reducing the pressure at a temperature of no less than 90°C. Thereafter, a polymerization reaction is allowed at 200 to 270°C to obtain polyglycerol. The polyglycerol thus obtained and the fatty acid are charged into a reaction vessel at an appropriate ratio, and thereto is added a sodium hydroxide solution as a catalyst. Next, the mixture is heated to a temperature of no less than 200°C under a nitrogen stream, and then a reaction is allowed for 1 to 3 hrs. Thereafter, the reaction is further allowed for 3 to 5 hrs at an internal temperature of no less than 250°C. Thereafter, after cooling to a normal temperature, the product is purified according to a common procedure, whereby a polyglycerol fatty acid ester can be obtained. It is to be noted that the HLB value of the polyglycerol fatty acid ester can be adjusted according to a common method.

Examples of the commercially available product of the polyglycerol fatty acid ester include "SUNSOFT A-186E", "SUNSOFT Q-175S", "SUNSOFT Q-185S", "SUNSOFT Q-17B", "SUNSOFT Q-18B", "SUNSOFT Q-18D", "SUNSOFT A-173E", "SUNSOFT Q-182S", "SUNSOFT Q-17S", "SUNSOFT A-171E", "SUNSOFT A-121E", "SUNSOFT Q-14S" manufactured by Taiyo Kagaku Co., Ltd., and the like.

In the oil or fat composition for use of the present invention, an appearance feature of the polyglycerol fatty acid ester is not particularly limited, and may be for example, oily, pasty, powdery, granular, or the like.

The polyglycerol fatty acid ester is an ester obtained from polyglycerol and a fatty acid derived from a natural vegetable oil, and has been conventionally used as an emulsifier for foods. The polyglycerol fatty acid esters have been conventionally accepted as food additives, and are very safe and devoid of a known side effect that may matter. In the oil or fat composition for use of the present invention, by selecting the polyglycerol fatty acid ester as an active ingredient, a side effect resulting from continuous ingestion can be avoided.

### [Oil or Fat Composition]

The triacylglycerol enables a plasma insulin level to be elevated by ingestion alone when a sugar (glucose) is ingested. In addition, the emulsifier enables a plasma insulin level to be elevated by use in combination with LCT when a sugar is ingested. To the contrary, according to the oil or fat composition for use of the present invention containing the triacylglycerol and the emulsifier as active ingredients, a synergistic effect of the triacylglycerol and the emulsifier enables a plasma insulin level to be prominently elevated.
Such an oil or fat composition for use of the present
invention effectively acts on animals including human suffering from diabetes or potential patients thereof who necessitate an increase in the amount of insulin secretion. In particular, the oil or fat composition for use of the present invention is suitable for the prevention or treatment of type II diabetes accompanied by insulin secretion failure that causes a decrease in the amount of insulin secretion. As referred to herein, the prevention means, for example, suppression and delay of the onset, and the like, whereas the treatment means, for example, delay progress, remission, alleviation and amelioration of symptoms, and the like.

A ratio (mass ratio) of the triacylglycerol to the emulsifier in the oil or fat composition for use of the present invention is preferably 99.5:0.5 to 98:2. When the ratio falls within the above range, an action of promoting insulin secretion that is effective in preventing diabetes and other uses can be exerted. It is to be noted that when two or more types of emulsifiers are contained, the total amount of these emulsifiers should fall within the above range of the ratio of the triacylglycerol to the emulsifier.

In the oil or fat composition for use of the present invention, various types of additives may be contained as needed within the range not to deteriorate the object of the present invention. Examples of the additive include an antioxidant, a defoaming agent, and the like. Examples of the antioxidant include tocopherols, tocotrienols, carotene, flavone, gallic acid, catechin and esters thereof, sesamol, terpenes, and the like. Examples of the defoaming agent include fine powder silica, silicone, and the like.

Since it is necessary to perform a pharmacological therapy for diabetes on a continuous basis for a long period of time, ingredients used in medical drugs are required to be devoid of concerns of side effects even if dosed continuously. Since the oil or fat composition for use of the present invention contains the triacylglycerol and the emulsifier described above that are very safe and devoid of concerns of side effects, as active ingredients, it can be reliably dosed on a continuous basis even when used in medical drugs. In addition, since dosing of the oil or fat composition for use of the present invention can be easily controlled, the composition is suited for pharmacological therapies including alimentary therapy in combination with exercise therapy carried out in the treatment of type II diabetes.

It was found that a synergistic insulin secretion effect is exhibited in the present invention, by using the triacylglycerol and the emulsifier in combination. Therefore, in the case in which the oil or fat composition for use of the present invention is used as a medical drug, it is possible to use the same in a state of a combination drug in which the triacylglycerol is mixed with the emulsifier, or in a state of a kit in which the triacylglycerol is not mixed with the emulsifier.

The administration route of the oil or fat composition for use of the present invention when used as a medical drug is preferably an oral administration route since the majority of the triacylglycerol and the emulsifier as active ingredients of the oil or fat composition for use of the present invention is absorbed into a living body through the mucosa of intestinal tract (small intestine). Examples of the formulation suitable for the oral administration include capsules, tablets, pills, powders, subtle granules, granules, liquids and solutions, syrups, and the like. The oil or fat composition for use of the present invention is preferably prepared into a formulation of a medical drug composition containing the triacylglycerol and the emulsifier added as active ingredients, and a pharmacologically and pharmaceutically acceptable additive. Examples of the pharmacologically and pharmaceutically acceptable additive include excipients such as glucose, lactose, crystalline cellulose and starch, disintegrants, binding agents, coating agents, coloring matters, diluents and the like, and a substance that is commonly used in formulation arts in general and does not react with the triacylglycerol and the emulsifier added as active ingredients may be used.

Since the oil or fat composition for use of the present invention is very safe and devoid of concerns of side effects due to continuous dosing as described above, the dosage amount of a preexisting drug can be lowered by use in combination, whereby side effect of the preexisting drug can be diminished. When used in combination with any other drug, the composition for use and the other drug may be included in a single pharmaceutical composition like a combination drug, or may be included in distinct pharmaceutical compositions.

The amount of administration of the medical drug prepared using the oil or fat composition for use of the present invention may be appropriately determined depending on a variety of conditions such as symptoms, age and body weight of the patient, the mode of administration, the administration duration, and the type of the treatment which may be prophylactic or therapeutic. For example, in the case in which therapies of diabetes of human (adult: 60 kg) are intended, the effective amount is usually 200 to 1,000 mg/kg of body weight/ day in terms of the triacylglycerol and 1 to 20 mg/kg of body weight/ day in terms of the emulsifier, and these doses may be administered once or divided for administration over several times, with appropriate adjustment that may be made so as to fall within the above range depending on the administration timing and the like. It is to be noted that the oil or fat composition for use of the present invention is effective if administered before or during meal.

The oil or fat composition for use of the present invention can be ingested as, for example, foods or drinks such as health foods and nutritional supplementary foods by packing into a soft capsule and/or processing. Also, the oil or fat composition for use of the present invention may be either directly ingested as is or after being processed into a powdery fat or a liquidified emulsion oil, or indirectly ingested by further processing these for use in general foods.

General foods in which the oil or fat composition for use of the present invention can be used are not particularly limit as long as they are processed food in which an oil or fat is used, and for example, breads and confectioneries such as breads, cakes, cookies, biscuits, doughnuts, muffins, scones, chocolates, gummy candies, snack foods, whipped creams and ice creams; drinks such as juice drinks, nutrition drinks and sports drinks; processed and seasoned foods such as soups, dressings, sources, mayonnaises, butters, margarines and prepared margarines; fat spreads, shortenings, bakery mixes, sautéing oils, frying oils, fried foods, processed meat products, frozen foods, noodles, retort foods, liquid diets, dysphagia diets, and the like may be exemplified.

In the case in which the oil or fat composition for use of the present invention is used for producing a food or drink for promoting insulin secretion targeting diabetic patients or potential patients thereof, for example, when prepared for adults (body weight: 60 kg), taking into consideration the type of the food or drink prepared, other ingredients included in the food or drink to be further ingested, and the like, it may be prepared such that the amount of the triacylglycerol becomes 200 to 1,000 mg/kg of body weight/ day and the amount of the emulsifier becomes 1 to 20 mg/kg of body weight/ day, which may be appropriately adjusted to fall within this range depending on the timing and the like of the ingestion.

### EXAMPLES

Hereinafter, the present invention will be explained in more detail by way of Examples.

### [Test Example 1] Study of Effect of Promoting Insulin Secretion (1)

Using rats subjected to carbohydrate load, an effect of promoting plasma insulin secretion by the oil or fat composition for use of the present invention (MLCT + emulsifier) was studied with LCT as a control.

### <Production Example 1> Method for Producing Sample 1

To 10 ml of a 40% glucose (D(+)-Glucose, manufactured by Wako Pure Chemical Industries, Ltd.) solution were added 2 g of LCT (trade name: Nisshin Canola Oil, manufactured by Nisshin OilliO Group, Ltd.) and 200 mg of bovine serum albumin (manufactured by SIGMA Co.), and the solution obtained was subjected to an ultrasonic treatment (5 min x 2, for 10 min in total) while cooling on ice to obtain Sample 1. A fatty acid composition of LCT (trade name: Nisshin Canola Oil, manufactured by Nisshin OilliO Group, Ltd.) used as a control is shown in Table 1.

**[Table 1]**

| Table 1 | |
|---|---|
| Fatty acid composition | Proportion (% by mass) |
| **C16:0** | **4.4** |
| **C18:0** | **2.1** |
| **C18:1** | **59.4** |
| **C18:2** | **20.7** |
| **C18:3** | **10.7** |
| Other long-chain fatty acid **(C20~C24)** | **2.7** |

### <Production Example 2> Method for Producing Sample 2

MCT (trade name: O.D.O, constituent fatty acids: n-octanoic acid/ n-decanoic acid = 3/1, manufactured by Nisshin OilliO Group, Ltd.), and LCT (trade name: Nisshin Canola Oil, manufactured by Nisshin OilliO Group, Ltd.) were mixed at a ratio of 14:86 (mass ratio) to give an oil mixture. To the oil mixture obtained was added a lipase powder in an amount of 0.1% by mass with respect to the oil mixture, and thereafter the mixture was stirred at 60^{Q}C for 15 hrs to allow for a transesterification reaction. Next, the lipase powder was filtered off from the reaction product, and the filtrate was washed with water and dried, followed by decolorization and deodorization to obtain a transesterified oil of MCT
with LCT (MLCT). Then, to 10 ml of a 40% glucose (D (+) - Glucose, manufactured by Wako Pure Chemical Industries, Ltd.) solution were added 2 g of the aforementioned MLCT, 20 mg of an emulsifier (trioleic pentaglycerol; trade name: SUNSOFT A-173E; HLB: 7; manufactured by Taiyo Kagaku Co., Ltd.) and 200 mg of bovine serum albumin (manufactured by SIGMA Co.), and the solution obtained was subjected to an ultrasonic treatment (5 min x 2, for 10 min in total) while cooling on ice to obtain Sample 2.

A triacylglycerol composition and a fatty acid composition of MLCT obtained in Production Example 2 are shown in Table 2 and Table 3, respectively. It is to be noted that the triacylglycerol composition was determined by gas chromatography using Silicone GS-1 manufactured by GL Sciences, Inc. as a column, and the fatty acid composition was determined in accordance with "Standard fat and oil analytical test methods (1996)".

| Table 2 | |
|---|---|
| Triacylglycerol composition | Proportion (% by mass) |
| **3M0L** | **0.5** |
| **2M1L** | **10.1** |
| **1M2L** | **36.2** |
| **0M3L** | **53.2** |
| **Total** | **100** |

| | |
|---|---|
| 3M0L: one molecule having 3 middle-chain fatty acid residues, and 0 long-chain fatty acid residues 2M1L: one molecule having 2 middle-chain fally acid residues, and 1 long-chain fally acid residue 1M2L: one molecule having 1 middle-chain fatty acid residue, and 2 long-chain fatty acid residues 0M3L: onz molecule having 0 middle-chain fatty acid residues, and 3 long-chain fatty acid residues | |

| Table 2 | |
|---|---|
| Triacylglycerol composition | Proportion (% by mass) |
| **3M0L** | **0.5** |
| **2M1L** | **10.1** |
| **1M2L** | **36.2** |
| **0M3L** | **53.2** |
| Total | **100** |
| 3M0L: one molecule having 3 middle-chain fatty acid residues, and 0 long-chain fatty acid residues | |
| 2M1 L: one molecule having 2 middle-chain fatty acid residues, and 1 long-chain fatty acid residue | |
| 1 M2L: one molecule having 1 middle-chain fatty acid residue, and 2 long-chain fatty acid residues | |
| 0M3L: one molecule having 0 middle-chain fatty acid residues, and 3 long-chain fatty acid residues | |

**[Table 3]**

| Table 3 | |
|---|---|
| Fatty acid composition | Proportion (% by mass) |
| **C8:0** | **8.8** |
| **C10:0** | **3.1** |
| **C16:0** | **2.9** |
| **C18:0** | **1.2** |
| **C18:1** | **57.0** |
| **C18:2** | **17.7** |
| **C18:3** | **7.2** |
| Other long-chain fatty acid **(C20~C24)** | **2.1** |

For the test, 7-weeks old male SD rats (purchased from Japan SLC, Inc.) were used. After the rats (17 animals) were kept for acclimation for one week, a carbohydrate tolerance test was conducted by a crossover study in which Sample 1
and Sample 2 were administered on the 8th week and 9th week. More specifically, in order to enable comparison of an effect achieved by MLCT and the emulsifier in an identical individual, a crossover study was employed in which the first carbohydrate tolerance test was followed by a wash-out period for one week, and then the second carbohydrate tolerance test was carried out. During the feeding period, individual rats were kept separately in each stainless mesh cage in an environment involving a temperature of 23 ± 1^{Q}C and a humidity of 50 ±
10%, under a 12/12-hour light-dark cycle (light: 8:00 to 20:00 h). Free intake of water and a solid animal feed (trade name: PicoLab Rodent Diet 20 5053, manufactured by Japan SLC, Inc.) was permitted. On the day before starting the test, the animals were starved from 17:00, and on the first day of the test, administration was started from 10:00.

The test was performed with two trials including "LCT administration trial (Sample 1 administration trial)" in which LCT was administered concomitantly with carbohydrate loading, and "(MLCT + emulsifier) administration trial (Sample 2 administration trial)" in which MLCT and the emulsifier were administered concomitantly with carbohydrate loading. Samples 1 and 2 were orally administered to the rats using a probe. The amount of the administration was 6 µl per gram of body weight of the rat. It is to be noted that the final amounts of the administration were 2 g of a glucide, 1 g of an oil and 0.01 g of the emulsifier per kg of body weight of the rat.

Before and 20 min after the administration, the blood was collected via the tail vein, and the plasma insulin level (ng/ml) was determined. It is to be noted that the plasma insulin level was determined according to an ELISA method using a commercially available kit (trade name: Rat Insulin ELISA kit, manufactured by Mercodia AB). The test results are shown in Fig. 1. The amount of an increase in the plasma insulin level in the (MLCT + emulsifier) administration trial was defined in terms of a relative value (%), when an average value of the amount of an increase in the plasma insulin level in the LCT administration trial was assumed to be 100, and represented by the average value ± standard error of the relative value. For a test of the significant difference between the trials, a Student t-test was employed, and the presence of the significant difference was decided with a significance level of less than 5%.

As shown in Fig. 1, the amount of an increase in the plasma insulin level of the (MLCT + emulsifier) administration trial exhibited a significantly (P < 0.001) high value as compared with the LCT administration trial. This revealed that the transesterified oil of MCT with LCT (MLCT) and the emulsifier promoted the secretion of insulin. It is to be noted that the average value (absolute value) of the amount of an increase in the plasma insulin level of the LCT administration trial was 1.13 ± 0.13 ng/ml, and the average value (absolute value) of the amount of an increase in the plasma insulin level of the (MLCT + emulsifier) administration trial was 2.28 ± 0.21 ng/ml.

### [Test Example 2] Study of Effect of Promoting Insulin Secretion (2) (Comparative)

Using rats subjected to carbohydrate load, an effect of promoting plasma insulin secretion by (LCT + emulsifier) was studied with LCT as a control.

### <Production Example 3> Method for Producing Sample 3

To 10 ml of a 40% glucose (D(+)-Glucose, manufactured by Wako Pure Chemical Industries, Ltd.) solution were added 2 g of LCT (trade name: Nisshin Canola Oil, manufactured by Nisshin OilliO Group, Ltd.), 20 mg of an emulsifier (trioleic pentaglycerol; trade name: SUNSOFT A-173E; HLB: 7; manufactured by Taiyo Kagaku Co., Ltd.) and 200 mg of bovine serum albumin (manufactured by SIGMA Co.), and the solution obtained was subjected to an ultrasonic treatment (5 min x 2, for 10 min in total) while cooling on ice to obtain Sample 3.

For the test, 7-weeks old male SD rats (purchased from Japan SLC, Inc.) were used. After the rats (17 animals) were kept for acclimation for one week, a carbohydrate tolerance test was conducted by a crossover study in which Sample 1 and Sample 3 were administered on the 8th week and 9th week. The feeding conditions were similar to Test Example 1.

The test was performed with two trials including "LCT administration trial (Sample 1 administration trial)" in which LCT was administered concomitantly with carbohydrate loading, and "(LCT + emulsifier) administration trial (Sample 3 administration trial)" in which LCT and the emulsifier were administered concomitantly with carbohydrate loading. Samples 1 and 3 were orally administered to the rats using a probe. The amount of the administration was 6 µl per gram of body weight of the rat. It is to be noted that the final amounts of the administration were 2 g of a glucide, 1 g of an oil and 0.01 g of the emulsifier per kg of body weight of the rat.

Before and 20 min after the administration, the blood was collected via the tail vein, and the plasma insulin level (ng/ml) was determined in a similar manner to Test Example 1. The test results are shown in Fig. 2. The amount of an increase in the plasma insulin level in the (LCT + emulsifier) administration trial was defined in terms of a relative value (%), when an average value of the amount of an increase in the plasma insulin level in the LCT administration trial was assumed to be 100, and represented by the average value ± standard error of the relative value. For a test of the significant difference between the trials, a Student t-test was employed, and the presence of the significant difference was decided with a significance level of less than 5%.

As shown in Fig. 2, the amount of an increase in the plasma insulin level of the (LCT + emulsifier) administration trial exhibited a significantly (P < 0.01) high value as compared with the LCT administration trial. However, when compared with the (MLCT + emulsifier) administration trial, the amount of an increase in the plasma insulin level was a significantly (P < 0.05) low value. It is to be noted that the average value (absolute value) of the amount of an increase in the plasma insulin level of the LCT administration trial was 1.32 ± 0.14 ng/ml, and the average value (absolute value) of the amount of an increase in the plasma insulin level of the (LCT + emulsifier) administration trial was 2.02 ± 0.20 ng/ml.

### [Test Example 3] Study of Effect of Promoting Insulin Secretion (3) (Comparative)

Using rats subjected to carbohydrate load, an effect of promoting plasma insulin secretion by MLCT was studied with LCT as a control.

### <Production Example 4> Method for Producing Sample 4

To 10 ml of a 40% glucose (D(+)-Glucose, manufactured by Wako Pure Chemical Industries, Ltd.) solution were added 2 g of MLCT obtained in Production Example 2 and 200 mg of bovine serum albumin (manufactured by SIGMA Co.), and the solution obtained was subjected to an ultrasonic treatment (5 min x 2, for 10 min in total) while cooling on ice to obtain Sample 4.

For the test, 7-weeks old male SD rats (purchased from Japan SLC, Inc.) were used. After the rats (19 animals) were kept for acclimation for one week, a carbohydrate tolerance test was conducted by a crossover study in which Sample 1 and Sample 4 were administered on the 8th week and 9th week. The feeding conditions were similar to Test Example 1.

The test was performed with two trials including "LCT administration trial (Sample 1 administration trial)" in which LCT was administered concomitantly with carbohydrate loading, and "MLCT administration trial (Sample 4 administration trial)" in which MLCT was administered concomitantly with carbohydrate loading. Samples 1 and 4 were orally administered to the rats using a probe. The amount of the administration was 6 µl per gram of body weight of the rat. It is to be noted that the final amounts of the administration were 2 g and 1 g of a glucide and an oil, respectively, per kg of body weight of the rat.

Before and 20 min after the administration, the blood was collected via the tail vein, and the plasma insulin level (ng/ml) was determined according to a similar method to Test Example 1. The test results are shown in Fig. 3. The amount of an increase in the plasma insulin level in the MLCT administration trial was defined in terms of a relative value (%), when an average value of the amount of an increase in the plasma insulin level in the LCT administration trial was assumed to be 100, and represented by the average value ± standard error of the relative value. For a test of the significant difference between the trials, a Student t-test was employed, and the presence of the significant difference was decided with a significance level of less than 5%.

As shown in Fig. 3, the amount of an increase in the plasma insulin level of the MLCT administration trial exhibited a significantly (P < 0.01) high value as compared with the LCT administration trial. However, when compared with the (MLCT + emulsifier) administration trial, the amount of an increase in the plasma insulin level was a significantly (P < 0.01) low value. It is to be noted that the average value (absolute value) of the amount of an increase in the plasma insulin level of the LCT administration trial was 0.86 ± 0.10 ng/ml, and the average value (absolute value) of the amount of an increase in the plasma insulin level of the MLCT administration trial was 1.15 ± 0.10 ng/ml.

### [Test Example 4] Study of Effect of Promoting Insulin Secretion (4) (Comparative)

Using rats subjected to carbohydrate load, an effect of promoting plasma insulin secretion by an oil mixture of MCT and LCT (MCT + LCT) was studied with LCT as a control.

### <Production Example 5> Method for Producing Sample 5

MCT (trade name: O.D.O, constituent fatty acids: n-octanoic acid/n-decanoic acid = 3/1, manufactured by Nisshin OilliO Group, Ltd.) and LCT (trade name: Nisshin Canola Oil, manufactured by Nisshin OilliO Group, Ltd.) were mixed at a ratio of 14:86 (mass ratio) to give an oil mixture of MCT and LCT (MCT + LCT). Then, to 10 ml of a 40% glucose (D(+)-Glucose, manufactured by Wako Pure Chemical Industries, Ltd.) solution were added 2 g of the (MCT + LCT), and 200 mg of bovine serum albumin (manufactured by SIGMA Co.), and the solution obtained was subjected to an ultrasonic treatment (5 min x 2, for 10
min in total) while cooling on ice to obtain Sample 5.

For the test, 7-weeks old male SD rats (purchased from Japan SLC, Inc.) were used. After the rats (14 animals) were kept for acclimation for one week, a carbohydrate tolerance test was conducted by a crossover study in which Sample 1 and Sample 5 were administered on the 8th week and 9th week. The feeding conditions were similar to Test Example 1.

The test was performed with two trials including "LCT administration trial (Sample 1 administration trial)" in which LCT was administered concomitantly with carbohydrate loading, and "(MCT + LCT) administration trial (Sample 5 administration trial)" in which the oil mixture of MCT and LCT was administered concomitantly with carbohydrate loading. Samples 1 and 5 were orally administered to the rats using a probe. The amount of the administration was 6 µl per gram of body weight of the rat. It is to be noted that the final amounts of the administration were 2 g and 1 g of a glucide and an oil, respectively, per kg of body weight of the rat.

Before and 20 min after the administration, the blood was collected via the tail vein, and the plasma insulin level (ng/ml) was determined according to a similar method to Test Example 1. The test results are shown in Fig. 4. The amount of an increase in the plasma insulin level in the (MCT + LCT) administration trial was defined in terms of a relative value (%), when an average value of the amount of an increase in the plasma insulin level in the LCT administration trial was assumed to be 100, and represented by the average value ± standard error of the relative value. For a test of the significant difference between the trials, a Student t-test was employed, and the presence of the significant difference was decided with a significance level of less than 5%.

As shown in Fig. 4, a significant difference was not found between the amount of an increase in the plasma insulin level of the (MCT + LCT) administration trial, and the amount of an increase in the plasma insulin level of the LCT administration trial. It is to be noted that the average value (absolute value) of the amount of an increase in the plasma insulin level of the LCT administration trial was 1.28 ± 0.18 ng/ml, and the average value (absolute value) of the amount of an increase in the plasma insulin level of the (MCT + LCT) administration trial was 1.25 ± 0.18 ng/ml.

From the results of Test Example 1 in the foregoing, it was proven that insulin secretion was promoted by the MLCT and the emulsifier. Also, from the results of Test Examples 3 and 4, the effect of promoting insulin secretion found when MLCT was used was revealed to result from the triacylglycerol having one or two medium-chain fatty acid residue(s) per molecule among the triacylglycerols included in MLCT. Furthermore, it was revealed from the results of Test Examples 1, 2, 3, and 4, that MLCT and the emulsifier both had an action of promoting insulin secretion; however, use of MLCT in combination with the emulsifier achieved a synergistic effect of promoting the insulin secretion.

### [Test Example 5] Study of Effect of Promoting Insulin Secretion (5)

Using diabetic model animals, an effect of promoting plasma insulin secretion by the oil or fat composition for use of the present invention (MLCT + emulsifier) was studied with LCT as a control.

For the test, 5 weeks old male ob/ob mice (purchased from Japan SLC, Inc.) were used. After the mice (10 animals) were kept for acclimation for one week, blood was collected at 9:00 a.m. to determine the blood glucose level. The blood glucose level was determined using Glucose C2 Test WAKO (manufactured by Wako Pure Chemical Industries, Ltd.). The animals were divided into two groups (five animals included in each group) such that each blood glucose level of each group was similar, and one group was employed as "LCT diet-ingested group (control group)" to which an animal feed containing LCT was given, and "(MLCT + emulsifier) diet-ingested group" to which an animal feed containing MLCT and the emulsifier was given. Formulations of the LCT feed and the (MLCT + emulsifier) feed used as test feeds are shown in Table 4. During the feeding period, individual rats were kept separately in each stainless mesh cage in an environment involving a temperature of 23 ± 1ºC and a humidity of 50 ± 10%, under a 12/12-hour light-dark cycle (light: 8:00 to 20:00 h). Free intake of water and the animal feed was permitted.

| Table 4 | | |
|---|---|---|
| Components | LCT diet (% by mass) | MLCT + emulsifier diet (% by mass) |
| corn starch | **36.7486** | **36.486** |
| milk casein | **20** | **20** |
| gelatinized corn starch | **13.2** | **13.2** |
| granulated sugar | **10** | **10** |
| MLCT + emulsifier* | **0** | **10** |
| LCT | **10** | **0** |
| cellulose powder | **5** | **5** |
| mineral mix | **3.5** | **3.5** |
| vitamin mix | **1** | **1** |
| L-cystine | **0.3** | **0.3** |
| choline bitartrate | **0.25** | **0.25** |
| tertiary butylhydroquinone | **0.0014** | **0.0014** |

| | | |
|---|---|---|
| *1% by mass emulsifier with respect to MLCT (trioleic pentaglycerol; trade name: SUNSOFT A-173E; HLB: 7; manufactured by Taiyo Kagaku Co., Ltd.) | | |

Feeding with the test feed was carried out for 3 weeks, and blood was collected from the tail vein once every morning at 9:00 am to determine the blood glucose level and the plasma insulin level. The plasma insulin level was determined using a Mouse Insulin ELISA kit (manufactured by Mercodia AB). With respect to the measurements of each group, mean and standard error were calculated. For a significance test, a two-way analysis of variance was employed using the feeding period and the fed animal feed as explanatory variables. For a multiple comparison test, a Turkey-Kramer method was employed. For the test, the presence of the significant difference was decided with a significance level of less than 5%. The results are shown in Fig. 5 ((A): casual blood glucose level, (B): plasma insulin level).

As shown in Fig. 5 (A), the blood glucose level gradually increased during the feeding period in the LCT diet-ingested group, whereas elevation of the blood glucose level was suppressed in the (MLCT + emulsifier) diet-ingested group, exhibiting a significantly (P < 0.05) low value of the blood glucose level as compared with the LCT diet-ingested group. Furthermore, as shown in Fig. 5 (B), the plasma insulin level in the (MLCT + emulsifier) diet-ingested group exhibited a significantly (P < 0.01) high value as compared with the LCT diet-ingested group.

From the foregoing results of Test Example 5, it was revealed that in the ob/ob mice that are diabetic model animals, ingestion of the animal feed containing MLCT and the emulsifier results in elevation of the plasma insulin level, and as a result, elevation of the blood glucose level is suppressed.

### INDUSTRIAL APPLICABILITY

The oil or fat composition for use of the present invention is effective in amelioration of conditions such as insulin secretion failure found in diabetic patients, and thus can be used in fields of medicine and food for diabetes.

## Claims

1. An oil or fat composition for use in treating or preventing diabetes, the composition comprising a triacylglycerol and an emulsifier as active ingredients, the triacylglycerol having one or two medium-chain fatty acid residue(s) in a molecule, and comprising a medium-chain fatty acid having 6 to 12 carbon atoms and a long-chain fatty acid having 16 to 24 carbon atoms as constituent fatty acids, and
the emulsifier being a polyglycerol fatty acid ester,
wherein a mass ratio of the triacylglycerol to the emulsifier in the oil or fat composition is 99.5:0.5 to 98:2.

2. The oil or fat composition for use according to claim 1, wherein the constituent fatty acids of the triacylglycerol include a medium-chain fatty acid having 6 to 12 carbon atoms and a long-chain fatty acid having 16 to 24 carbon atoms.

3. The oil or fat composition for use according to claim 1 or 2, wherein the medium-chain fatty acid is at least one selected from the group consisting of a saturated fatty acid having 8 carbon atoms and a saturated fatty acid having 10 carbon atoms.

4. A food or drink composition comprising the oil or fat composition according to any one of claims 1 to 3 for use according to any one of claims 1 to 3.

## Patentansprüche

1. Fett- oder Ölzusammensetzung zur Verwendung in der Behandlung oder Vorbeugung von Diabetes, wobei die Zusammensetzung ein Triacylglycerol und einen Emulgator als Wirkstoffe beinhaltet, wobei das Triacylglycerol einen oder zwei mittelkettige(n) Fettsäurerest(e) in einem Molekül aufweist, und umfassend eine mittelkettige Fettsäure mit 6 bis 12 Kohlenstoffatomen und eine langkettige Fettsäure mit 16 bis 24 Kohlenstoffatomen als Bestandteil-Fettsäuren, und
wobei der Emulgator ein Polyglyzerinfettsäureester ist,
worin ein Massenverhältnis von Triacylglycerol zum Emulgator in der Fett- oder Ölzusammensetzung 99.5:0.5 bis 98:2 ist.

2. Fett- oder Ölzusammensetzung zur Verwendung gemäss Anspruch 1, worin die Bestandteil-Fettsäuren des Triacylglycerols eine mittelkettige Fettsäure mit 6 bis 12 Kohlenstoffatomen und eine langkettige Fettsäure mit 16 bis 24 Kohlenstoffatomen beinhalten.

3. Fett- oder Ölzusammensetzung zur Verwendung gemäss Anspruch 1 oder 2, worin die mittelkettige Fettsäure mindestens eine ist ausgewählt aus der Gruppe bestehend aus: einer gesättigten Fettsäure mit 8 Kohlenstoffatomen und einer gesättigten Fettsäure mit 10 Kohlenstoffatomen.

4. Lebensmittelzusammensetzung oder Getränke mit der Fett- oder Ölzusammensetzung gemäss irgendeinem der Ansprüche 1 bis 3 zur Verwendung gemäss irgendeinem der Ansprüche 1 bis 3.

## Revendications

1. Composition d'huile ou de graisse pour son utilisation dans le traitement ou la prévention du diabète, la composition comprenant un triacylglycérol et un émulsifiant comme ingrédients actifs, le triacylglycérol ayant un ou deux résidu(s) d'acide gras à chaîne moyenne dans une molécule, et comprenant un acide gras à chaîne moyenne ayant 6 à 12 atomes de carbone et un acide gras à chaîne longue ayant 16 à 24 atomes de carbone comme acides gras constitutifs, et
l'émulsifiant étant un ester d'acide gras polyglycérol,
dans lequel un rapport de masse du triacylglycérol par rapport à l'émulsifiant dans la composition d'huile ou de graisse est 99.5:0.5 à 98:2.

2. Composition d'huile ou de graisse pour son utilisation selon la revendication 1, dans laquelle les acides gras constitutifs du triacylglycérol comprennent un acide gras à chaîne moyenne ayant 6 à 12 atomes de carbone et un acide gras à chaîne longue ayant 16 à 24 atomes de carbone.

3. Composition d'huile ou de graisse pour son utilisation selon la revendication 1 ou 2, dans laquelle l'acide gras à chaîne moyenne est au moins un sélectionné parmi le groupe consistant en un acide gras saturé ayant 8 atomes de carbone et un acide gras saturé ayant 10 atomes de carbone.

4. Composition alimentaire ou boisson comprenant la composition d'huile ou de graisse selon l'une quelconque des revendications 1 à 3 pour son utilisation selon l'une quelconque des revendications 1 à 3.
